# EUROPEAN PATENT APPLICATION

(11) **EP 4 674 945 A1**
(43) Date of publication of application: **07.01.2026**
(21) Application number: 24771172.4
(22) Date of filing: 12.03.2024
(51) Int. Cl.: C12M 3/00, C12M 1/36, C12M 1/26, C12M 1/00, C12M 1/34, C12M 1/32, C12M 3/06

(54) **CARTRIDGE FOR ADHERENT CELL CULTURE, AND CELL CULTURE DEVICE INCLUDING SAME**

(30) Priority: 15.03.2023 KR 20230033845; 11.01.2024 KR 20240004952; 21.02.2024 KR 20240024811
(71) Applicant: Celloid Co., Ltd., Pohang-si, Gyeongsangbuk-do 37673 (KR)
(72) Inventor: MIN, Byung Hyuk, Pohang-si, Gyeongsangbuk-do 37673 (KR); LEE, Donghyeon, Seoul 04570 (KR); LEE, Seong Jin, Pohang-si, Gyeongsangbuk-do 37665 (KR); KIM, Dong Sung, Pohang-si, Gyeongsangbuk-do 37673 (KR); BANG, Jun Yong, Pohang-si, Gyeongsangbuk-do 37673 (KR)
(74) Representative: BCKIP Part mbB
(86) International application number: PCT/KR2024/003178
(87) International publication number: WO 2024/191173

(57) **Abstract**

The present invention relates to a cartridge for adherent cell culture and a cell culture device including same and, more particularly, to a device implemented to automate a culture process of adherent cells, and a cartridge that is attachable to/detachable from the device.

## Description

### Technical Field

The present invention relates to a cartridge for adherent cell culture and a cell culture device including the same, more particularly to a device implemented to automate a culture process of adherent cells and a cartridge that is detachably attachable thereto.

### Background Art

Cell culture is a technology necessary in research and development for functions and characteristics of cells, diseases, and treatment in various fields. However, the cell culture generally takes many hours and significant effort, and since risk factors such as contamination, transition, and the like are numerous in the process of cell culture, the cell culture requires a sophisticated culture environment. Further, a quality of cell culture and reproducibility in cell culture are heavily dependent on a worker's skill and manipulation technique, and therefore, it is hard to ensure reliability and consistency in test results. Under such a situation, recently, a need for automating cell culture has increased greatly, and there are not a few companies that develop a technology for automating cell culture.

The present invention is provided to overcome the above-mentioned problems and thus characterized in that an environment where culture media for cell growth are automatically changed in culture spaces for culturing adherent cells is automatedly built. Further, the present invention is provided with additional technical components that would not have been obvious to one of ordinary skill in the art.

The present invention has the support of national research and development projects as will be listed below.
[Subject unique No.] 1415187257
[Subject No.] 20023762
[Ministry name] Ministry of Trade, Industry and Energy
[Subject management (professional) organization name] Korea Planning & Evaluation Institute of Industrial Technology
[Research business name] Industrial technology development of machinery and equipment
[Research subject name] Development of 3D bioreactor automation culture system for in situ uniform cell coagulation, proliferation and differentiation based on material-transmitting thin film scaffold with a solid concave-convex structure and permeable ultrafine perfusion
[Subject lead organization name] Celloid Co. Ltd.
[Research period] April 1, 2023 to December 31, 2026
[Subject unique No.] 1425178283
[Subject No.] S3295878
[Ministry name] Ministry of SMEs and Startups
[Subject management (professional) organization name] Korea technology & information promotion agency for SMEs
[Research business name] Technological development for startup growth
[Research subject name] Development for organoid culture automation system capable of having uniformity and high reproducibility, based on highly permeable nanofibrous microwell
[Subject lead organization name] Celloid Co. Ltd.
[Research period] July 1, 2022 to June 30, 2024

### Disclosure

### Technical Problem

It is an object of the present invention to provide a cell culture device that is capable of automating a culture process of adherent cells.

It is another object of the present invention to provide a cell culture device that is capable of allowing a cartridge detachable therefrom to be utilized as cell culture spaces, thereby being used conveniently by a user and preventing contamination and transition of cells from occurring during cell culture.

The technical problems to be achieved through the present invention are not limited as mentioned above, and other technical problems not mentioned herein will be obviously understood by one of ordinary skill in the art through the following description.

### Technical Solution

To accomplish the above-mentioned object, according to one aspect of the present invention, a cartridge for adherent cell culture may include: one or more culture spaces; a culture media adjusting part for adjusting the injection of culture media into the culture spaces; culture media feed channels for moving the culture media to the culture media adjusting part from the outside; and culture media injection channels extending from the culture media adjusting part to move the culture media to the culture spaces, wherein the culture media adjusting part may include a connector adapted to branch the culture media fed from the culture media feed channels into the culture media injection channels.

Further, the cartridge for adherent cell culture may further include actuators adapted to control the injection of the culture media moving through the culture media injection channels.

Furthermore, the actuators of the cartridge for adherent cell culture may press the culture media injection channels made of a flexible material so that the injection of the culture media may be controlled.

Moreover, the culture media injection channels of the cartridge for adherent cell culture may be made of the flexible material and consist of first injection channels pressed against the culture media adjusting part in such a way to adjust the feed of the culture media and second injection channels extending from the first injection channels to feed the culture media to the culture spaces.

Besides, the actuators of the cartridge for adherent cell culture may remain in a non-pressing state where the first injection channels are not pressed thereagainst only while external forces are applied thereto, whereas the actuators may press the first injection channels thereagainst while external forces are not applied thereto.

Further, the actuators of the cartridge for adherent cell culture may remain in a pressing state where the first injection channels are pressed thereagainst only while external forces are applied thereto, whereas the actuators may not press the first injection channels thereagainst while external forces are not applied thereto.

Furthermore, the actuators of the cartridge for adherent cell culture may change a state where the first injection channels are pressed thereagainst only while external forces are applied thereto, whereas the actuators may remain in a pressing state where the first injection channels are pressed thereagainst while external forces are not applied thereto.

Further, at least some of the culture media feed channels of the cartridge for adherent cell culture may move different types of culture media from other culture media feed channels.

Furthermore, the cartridge for adherent cell culture may have one culture space, extend the plurality of culture media injection channels from the culture media adjusting part, and feed the culture media to the culture space through the culture media injection channels.

Additionally, the components constituting the cartridge for adherent cell culture may be made of only polymers or metals.

Further, the cartridge for adherent cell culture may further include
a lid for covering a whole of the culture spaces so that the culture media may be injected into the culture spaces by means of the culture media injection channels passing through the lid.

The lid may be designed to receive an external force from the culture media injection channels passing through tops of the lids, and the culture media injection channels may apply the external force to the lid so that the culture spaces may be closed with the lid.

To accomplish the above-mentioned object, according to another aspect of the present invention, a device for automating cell culture may include: a cartridge detachably mounted thereon and including: one or more culture spaces; a culture media adjusting part for adjusting the injection of culture media into the culture spaces; culture media feed channels for moving the culture media to the culture media adjusting part from the outside; and culture media injection channels extending from the culture media adjusting part to move the culture media to the culture spaces; a pressing part for applying an external force to the culture media adjusting part; and a controller for controlling the pressing part.

Further, the controller of the device for automating cell culture may feed the culture media to the culture media injection channels sequentially and repeatedly for a predetermined time, while controlling the pressing part.

Furthermore, the cartridge of the device for automating cell culture may further include a lid for covering a whole of the culture spaces so that the culture media may be injected into the culture spaces by means of the culture media injection channels passing through the lid.

Moreover, the cartridge of the device for automating cell culture may further include a fastener for detachably coupling the device for automating cell culture thereto.

### Advantageous Effects of Invention

According to the present invention, the cell culture device provides environments in which the culture media for cell growth are automatedly replaced in the plurality of individual culture spaces, thereby minimizing occurrence of contamination or transition of cells to ensure the culture of good quality cells.

According to the embodiment of the present invention, further, the cell culture device builds a cell culture environment according to a sophisticated mechanism, thereby ensuring stable cell culture.

According to the embodiment of the present invention, furthermore, the cell culture device is intuitively manipulated even by a user who does not have enough experience, and since qualities of adherent cells cultured are not influenced by the user's experience, anybody can easily perform the cell culture.

The effectiveness of the invention is not limited as mentioned above, and it should be understood to those skilled in the art that the effectiveness of the invention may include another effectiveness as not mentioned above from the detailed description of the present invention.

### Brief Description of Drawings

FIG. 1 shows a cell culture device in which a cartridge is mounted according to the present invention.
FIG. 2 shows the cartridge according to an embodiment of the present invention.
FIG. 3 shows components of a culture media adjusting part of the cartridge according to the present invention.
FIGs. 4a to 4c show the cartridge with a culture media storage part and an example of a tray of the cartridge.
FIGs. 5 and 6 show the principle in which the injection of culture media is controlled in the culture media adjusting part.
FIG. 7 shows an example of a lid for covering culture spaces.
FIG. 8 shows a portion of the cartridge designed to allow the lid to be kept open.
FIGs. 9a to 9b show the lid designed to receive a force from culture media injection channels made of a flexible material so that the lid covers the culture spaces.
FIGs. 10a and 10b show the components of the cell culture device according to the present invention.
FIGs. 11a and 11b show a coupled relation between the cartridge and the cell culture device when the cartridge is mounted on the cell culture device.
FIGs. 12a and 12b show a monitoring part of the cell culture device according to the present invention.

### Mode for Invention

Hereinafter, an embodiment of the present invention will be described with reference to the accompanying drawings. Objects, characteristics and advantages of the present invention will be more clearly understood from the detailed description as will be described below and the attached drawings. Before the present invention is disclosed and described, it is to be understood that the disclosed embodiments are merely exemplary of the invention, which can be embodied in various forms. Therefore, specific structural and functional details disclosed herein are not to be interpreted as limiting, but merely as a basis for the claims and as a representative basis for teaching one of ordinary skill in the art to variously employ the present invention in virtually any appropriately detailed structure. The corresponding parts in the embodiments of the present invention are indicated by corresponding reference numerals.

All terms used herein, (including technical or scientific terms), unless otherwise defined, have the same meanings which are typically understood by those having ordinary skill in the art. The terms, such as ones defined in common dictionaries, should be interpreted as having the same meanings as terms in the context of pertinent technology, and should not be interpreted as having ideal or excessively formal meanings unless clearly defined in the specification. An expression referencing a singular value additionally refers to a corresponding expression of the plural number, unless explicitly limited otherwise by the context.

Terms, such as the first, the second, and the like may be used to describe various elements, but the elements should not be restricted by the terms. The terms are used to only distinguish one element from the other element. For example, a first element may be named a second element without departing from the scope of the present invention.

In this application, terms, such as "comprise", "include", or 'have", are intended to designate those characteristics, numbers, steps, operations, elements, or parts which are described in the specification, or any combination of them that exist, and it should be understood that they do not preclude the possibility of the existence or possible addition of one or more additional characteristics, numbers, steps, operations, elements, or parts, or combinations thereof.

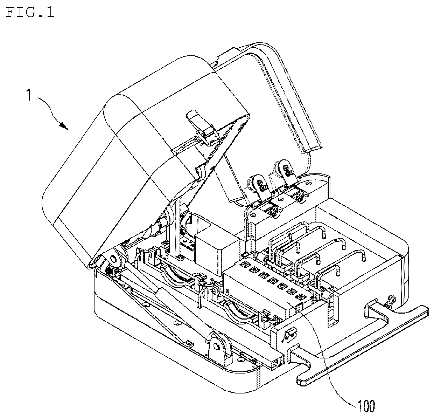

FIG. 1 is a perspective view showing a cell culture device 1 and a cartridge 100 according to the present invention, and as shown, the cartridge 100 is mounted in the cell culture device 1. As mentioned above, the cell culture device 1 of the present invention is adapted to automate cell culture so that injection of culture media is automatedly controlled and monitoring for cells cultured is automatedly controlled.

According to one of the characteristics of the present invention, further, the cell culture device 1 is designed to allow the cartridge 100 to be detachably attached thereto, so that in a state where the cartridge 100 is detached from the cell culture device 1, a user can perform a plurality of tasks (for example, cell injection, cell monitoring, and so on), and as the cartridge once used is thrown away and a new cartridge is required, a clean state in which a cell culture environment is not contaminated is kept.

First, the cartridge 100 of the present invention will be explained, and next, the cell culture device 1 in which the cartridge 100 is mounted will be described later.

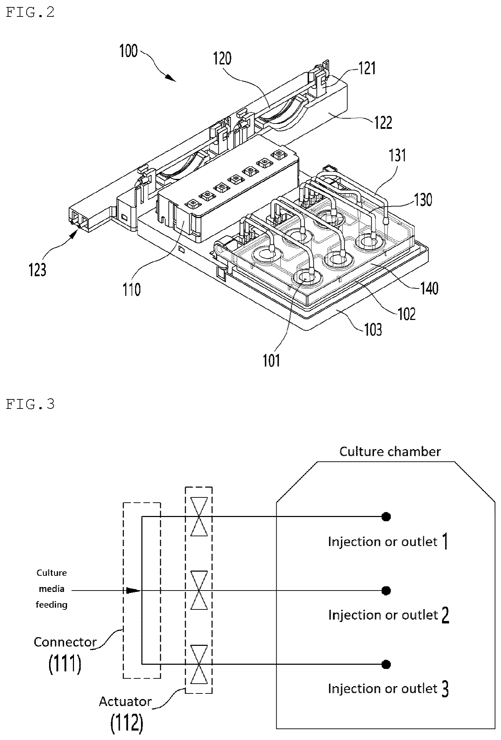

FIG. 2 shows components of the cartridge 100. As shown, the cartridge 100 largely includes culture spaces 101, a culture media adjusting part 110, culture media feed channels 120, culture media injection channels 130, and a lid 140.

The culture spaces 101 mean spaces in which adherent cells are cultured, and only if the culture spaces 101 are spaces in which culture media are fed to cells to allow the cells to appropriately grow, they may not be limited in shape or structure. Desirably, however, the culture spaces 101 are defined on a tray 102 located on a tray stand 103, as shown, and the shapes or volumes of the culture spaces 101 are defined according to a design structure of the tray 102 when the tray 102 is made. In the description of the present invention, furthermore, six culture spaces, which are separated from one another, are defined on the tray 102, but of course, only one culture space may exist on the tray 102. Even though only one culture space exists, the plurality of culture media injection channels 130 as shown are located above the corresponding culture space to mixedly feed different types of culture media to the culture space.

Next, the culture media adjusting part 110 serves to adjust the injection of the culture media fed to the culture spaces 101. The culture media adjusting part 110 of the cartridge 100 is located at a position for connecting the culture media feed channels 120 and the culture media injection channels 130, and further, it is designed to allow the fed culture media to be branchedly fed to the culture spaces 101 through the culture media injection channels 130. The culture media adjusting part 110 largely includes two functional components, that is, a connector 111 and actuators 112.

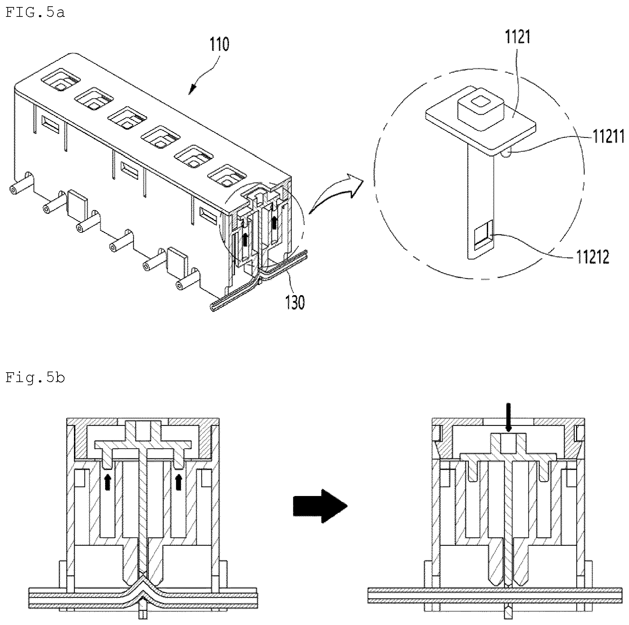

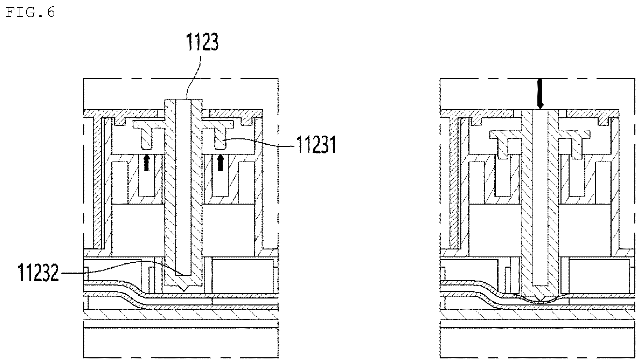

Referring to FIG. 3, the connector 111 serves to allow the culture media fed to be branched into the culture media injection channels 130, and the actuators 112 serve to control whether the culture media moving along the culture media injection channels 130 are injected or not. The connector 111 is formed of a pipe or tube structure having one inlet and a plurality of outlets, and the actuators 112 are mechanisms for closing or opening the flows of media in the channels (pipes, tubes, etc.) through which the culture media pass. In the drawings, the culture media fed to the culture media adjusting part 110 are branched into three channels by means of the connector 111, and the flows of the branched culture media are controlled by means of the actuators 112 through which the respective channels pass. The actuators 112 will be explained again with reference to FIGs. 5 and 6 as will be described later.

It is noted that the cartridge 100 of the present invention is a structure in which electric power is not generated by itself or no electric power is supplied thereto, but without being limited thereto, the cartridge 100 may operate with electric power if necessary. It is therefore appreciated that the actuators 112 make use of the electric power received thereto to thus determine whether the culture media are fed to the culture media injection channels 130.

Referring back to FIG. 2, the cartridge 100 is provided with the culture media feed channels 120. In this case, of course, a plurality of culture media feed channels 120, not one culture media feed channel 120, are provided to feed different types of culture media at a time. Further, the culture media feed channels 120 are desirably formed of a pipe or tube, more desirably made of a flexible material. As shown, the culture media feed channels 120 are fixed to culture media feed channel support stands 121 located on culture media feed channel support plates 122, and a semicircular groove is formed between the neighboring culture media feed channel support stands 121 in such a way as to engage with a pump of the cell culture device 1 as will be discussed later. Further, the culture media feed channels 120 extend from culture media feed pipes 123, and the culture media feed pipes 123 are connected to a culture media storage part not shown in the drawings and thus serve to feed the culture media to the culture media feed channels 120.

As shown, the culture media injection channels 130 extend from the culture media adjusting part 110 to feed the culture media to the culture spaces 101. Like the culture media feed channels 120, the culture media injection channels 130 are desirably formed of a pipe or tube and made of a flexible material. Further, each culture media injection channel 130 is divided into a portion located inside the culture media adjusting part 110 and a portion located outside the culture media adjusting part 110, and if the channel located inside the culture media adjusting part 110 is defined as a first injection channel and the channel located outside the culture media adjusting part 110 as a second injection channel, at least the first injection channel is made of the flexible material to allow the flow of the culture media to be easily controlled in the culture media adjusting part 110. Furthermore, culture media discharge channels 131 are additionally provided if necessary, and besides, channels for feeding or discharging different types of media (oxygen, carbon dioxide, water, and other gases or liquids) to or from the culture spaces 101, not for feeding or discharging the culture media, may be additionally provided.

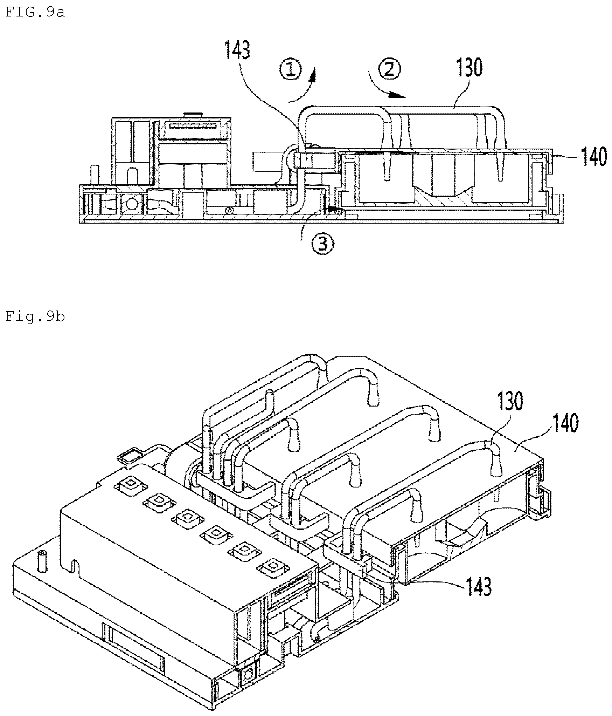

The lid 140 serves to cover a whole of the culture spaces 101 or a portion thereof and has through holes along which the culture media pass and are thus fed to the respective culture spaces 101. The through holes are connected to the culture media injection channels 130, and otherwise, the culture media injection channels 130 directly pass through the through holes, thereby allowing the culture media to come into close contact with the culture spaces 101. Further, the cartridge 100 of the present invention is designed to apply an external force to the lid 140 so that the lid 140 basically covers the culture spaces 101, and in this case, the external force is generated due to the culture media injection channels 130 made of the flexible material. This will be explained in detail with reference to FIGs. 9a to 9b as will be discussed later.

The components constituting the cartridge 100 of the present invention have been described with reference to FIG. 2.

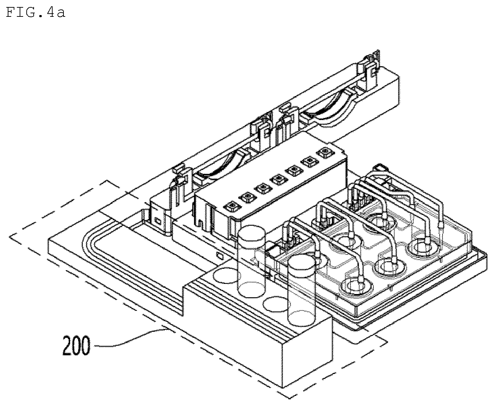

FIGs. 4a to 4c show a variation of the cartridge 100, an example of the tray, and an example in which the culture media injection channels 130 pass through the lid 140. FIG. 4a shows an example in which a culture media storage part 200 is additionally provided for the above-mentioned cartridge 100. In the above description, the culture media feed channels 120 are connected to the culture media feed pipes 123, and in this case, as shown, the culture media feed pipes 123 are desirably connected to the culture media storage part 200, more particularly to culture media storage containers to feed the culture media.

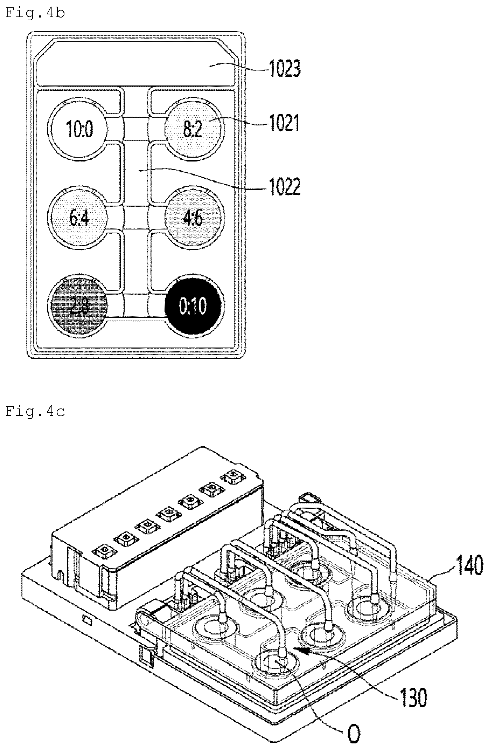

FIG. 4b shows the tray 102 that is located in the cartridge 100 to perform cell culture, and the tray 102 includes cell culture areas 1021 into which cells are injected and thus grow, a media collection space 1023 for collecting the culture media flowing from the cell culture areas 1021, and a media moving path 1022 along which the culture media flow from the cell culture areas 1021 to the media collection space 1023. The term, culture spaces 101, as used herein, means the cell culture areas 1021, in a narrow sense, but means all areas needed for cell culture, that is, the areas from the reference numerals 1021 to the reference numeral 1023, in a broad sense. The tray 102 as shown in FIG. 4b has six different cell culture areas 1021, that is, the plurality of culture spaces 101, and in this case, it can be appreciated that the respective culture spaces 101 are adjusted in concentration of the culture media, independently of one another (wherein ratios as indicated on the respective cell culture areas mean ratios of culture media to water) and through such independent space division, the cell culture can be performed in different environments. As shown in FIG. 2, the culture media injection channels 130 are connected to the respective culture spaces 101, and since the culture media are fed according to the different adjustment methods through the culture media injection channels 130, the plurality of divided independent culture environments as shown in FIG. 4b can be built.

FIG. 4c shows the positions of the culture media injection channels 130 passing through the culture spaces 101 around the centers O of the culture spaces 101 when the culture media injection channels 130 pass through the lid 140 and the culture spaces 101 sequentially to perform the injection of the culture media. According to an embodiment of the present invention, the positions of the culture media injection channels 130 passing through the culture spaces 101, more particularly the positions of injection portions of the culture media injection channels 130 on the plane may correspond to the centers O of the culture spaces 101, but desirably, the injection portions of the culture media injection channels 130 are located on the peripheries of the culture spaces 101, not on the centers O thereof. More desirably, the injection portions of the culture media injection channels 130 are designed to be located on the outer boundaries of the culture spaces 101 to allow the culture media dropped or dripped therefrom to flow along the inner walls of the culture spaces 101. The reason why the injection portions of the culture media injection channels 130 are deviated from the centers O of the culture spaces 101 is because the cells inside the culture spaces 101 are protected from impacts generated when the culture media are dropped or dripped, the culture media collected in the culture spaces 101 by means of dropping or dripping are prevented from splashing in every direction, and the loss of the cells growing in the culture spaces 101 is minimized.

FIGs. 5a, 5b and 6 show the actuating principle of the culture media adjusting part 110, more particularly the actuators 112.

First, FIGs. 5a and 5b show a design structure in which a mechanism is provided to prevent the culture media injection channels 130 from being compressed, while external forces are applied to the actuators 112, and to allow the culture media injection channels 130 to be compressed, while no external forces are applied to the actuators 112. To do this, the culture media adjusting part 110 (more particularly each actuator 112) has a first cylinder 1121, and the first cylinder 1121 consists of spring supporters 11211 for receiving elasticity of a spring to allow the first cylinder 1121 to move up and down and a tube compressor 11212 having the shape of a hole through which the corresponding culture media injection channel 130 passes in such a way as to open or close the flow of the culture media of the corresponding culture media injection channel 130 according to the upward and downward movements of the first cylinder 1121. The left figure of FIG. 5b shows a state wherein no external force is applied to the culture media adjusting part 110, and in this case, the first cylinder 1121 moves upward by means of the spring so that the corresponding culture media injection channel 130 is closed by the tube compressor 11212. Contrarily, the right figure of FIG. 5b shows a state wherein an external force is applied to the culture media adjusting part 110, and in this case, the first cylinder 1121 moves downward in a direction opposite to the applied direction of the elasticity of the spring so that the corresponding culture media injection channel 130 is open.

FIG. 6 shows another example of the actuator 112, which is different from the actuator 112 as shown in FIGs. 5a and 5b. That is, FIG. 6 shows a design structure in which a mechanism is provided to allow the culture media injection channels 130 to be compressed, while external forces are applied to the actuators 112, and to prevent the culture media injection channels 130 from being compressed, while no external forces are applied to the actuators 112. As shown, second cylinders 1123 are located in the culture media adjusting part 110, and in this case, each second cylinder 1123 has spring supporters 11231 having the similar shape to that of the first cylinder 1121. However, the second cylinder 1123 has a tube compressor 11232 configured differently from that of the first cylinder 1121. In detail, the tube compressor 11232 of the second cylinder 1123 is designed to compress the corresponding culture media injection channel 130 against the end portion thereof.

Even though not shown in the drawings, the actuators 112 of the culture media adjusting part 110 may be designed to allow the culture media injection channels 130 to be compressed, while the external forces are applied thereto, and to allow the culture media injection channels 130 to be kept in the compressed states, while the external forces are not applied thereto.

As shown in FIGs. 5 and 6, further, the culture media injection channels 130 as compression targets are located inside the culture media adjusting part 110. That is, it can be appreciated that they are the first injection channels as mentioned above.

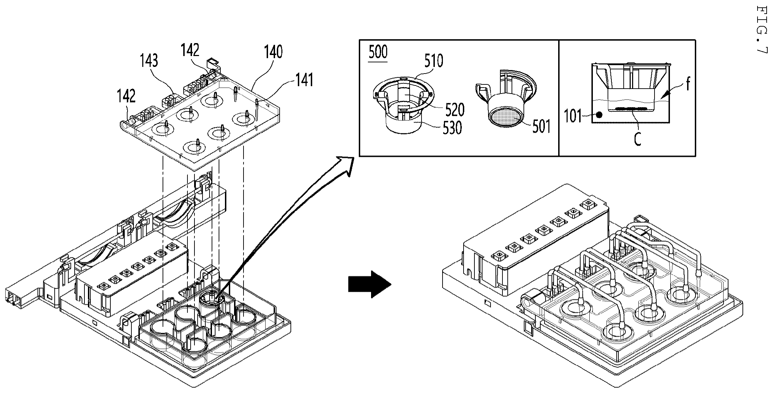

FIGs. 7 to 9 show a structure in which the lid 140 is coupled to the cartridge 100 and a mechanism applied when the lid 140 is open and closed through the coupling.

FIG. 7 shows the components of the lid 140 and a state wherein the lid 140 is coupled to the cartridge 100. The lid 140 desirably has channel connection protrusions 141 protruding from top thereof in such a way as to fittedly connect one end of culture media injection channels 130 thereto, lid fixing shafts 142 located on the rear side thereof in such a way as to be coupled to the cartridge 100 and thus become portions of rotary shafts, and channel fixing stands 143 for fittedly fixing the culture media injection channels 130 thereto. The right side of FIG. 7 shows states in which the lid 140 is coupled to the cartridge 100 and thus closed and the culture media injection channels 130 are firmly fitted to the channel fixing stands 143.

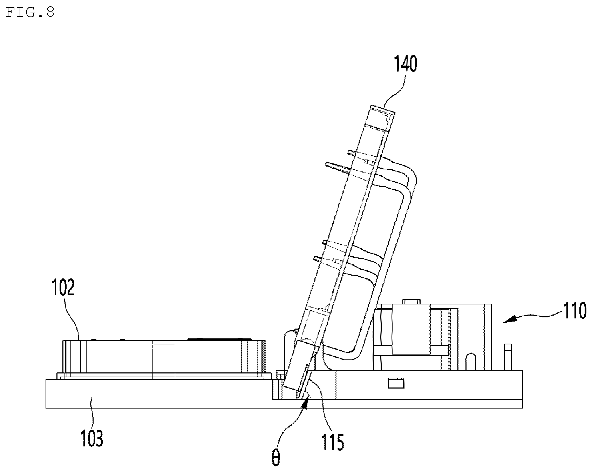

FIG. 8 shows a state in which the lid 140 is open, and as shown, the lid 140 is open to a given angle by means of a reclining prevention projection 115, without completely falling backward. Exactly, the reclining prevention projection 115 is a component of the culture media adjusting part 110, but to allow the lid 140 to be kept open at the given angle, another structure may be provided.

FIGs. 9a to 9b show a state in which the lid 140 receives a force in a given direction so as to cover the culture spaces 101, that is, a state wherein a force is applied in a given direction so that the lid 140 covers the culture spaces 101 by means of the restoring forces of the culture media injection channels 130 made of the flexible material.

The culture media injection channels 130 made of the flexible material, more accurately the flexible tubes have the restoring forces to their original state when bent, and as shown in FIG. 9a or 9b, if the culture media injection channels 130 having the restoring forces are fixed in position by means of the channel fixing stands 143, the forces (forces Nos. ① and ②) applied to a direction so that the lid 140 is open are offset by means of the existences of the channel fixing stands 143, and therefore, the restoring forces of the culture media injection channels 130 made of the flexible material are applied only to a force (force No. ③) applied to a direction so that the lid 140 is closed, thereby allowing the closed state as shown in FIGs. 9a to 9b to be kept. As shown, it can be checked that in a state where the flexible tubes are connected to the lid 140, they are bent on portions indicated with the forces Nos. ③, ① and ②.

In building the cell culture environments, it is very important to prevent pollutants from entering the culture spaces 101, and to do this, the cartridge 100 of the present invention allows the culture media injection channels 130 extending from the culture media adjusting part 110 to be made of the flexible material, allow the culture media injection channels 130 to pass through the lid 140, and allow the channel fixing stands 143 located on the rear side of the lid 140 to fix the culture media injection channels 130 thereto to offset the restoring forces of the culture media injection channels 130 toward the direction for opening the lid 140 so that the lid 140 is always kept closed by means of the restoring forces of the flexible tubes.

The components of the cartridge 100 of the present invention have been explained in detail with reference to the attached drawings.

Further, FIG. 7 shows an insert 500 having an insertable structure into the corresponding culture space 101. According to the present invention, the cell culture device 1 is configured to allow the culture spaces 101 to be formed on the tray 102 so that cells and media are fed to and injected into the respective culture spaces 101, thereby performing the cell culture. According to a desirable embodiment of the present invention, further, the inserts 500 as shown are inserted into the corresponding culture spaces 101 of the cell culture device 1, thereby achieving more improved cell culture environments. As shown, each insert 500 includes an extending frame 510 laid on the peripheral wall of the corresponding culture space 101, a vertical frame 520 extending vertically from the extending frame 510, and a culture frame 530 extending from the vertical frame 520 to form a space in which cells or tissues are located. Further, the insert 500 has a bottom membrane 501 located on the bottom of the culture frame 530 in such a way as to allow media to pass therethrough, while preventing the cells or tissues from passing therethrough.

FIG. 7 shows a state wherein a cell culture environment in which the cells c are located on top of the bottom membrane 501 and a surface f of the culture media is lower than the uppermost end of the culture frame 530 is built in a state where the insert 500 is inserted into the corresponding culture space 101. Like this, the existence of the insert 500 enables the cells to be cultured to grow on top of the bottom membrane 501, not on the bottom of the culture space 101, that is, not on the bottom of the tray 102, thereby allowing materials to move and diffuse smoothly to thus provide positive effectiveness for the cell culture.

FIG. 7 shows the state wherein the bottom membrane 501 is provided for the culture space 101 only under the condition of the existence of the insert 500, but only if the bottom membrane 501 is located in the culture space 101 to allow the cells or tissues to grow thereon, while the cells or tissues are not brought into direct contact with the bottom of the culture space 101, it will be appreciated that a shape of the bottom membrane 501 existing in the culture space 101 and a structure in which the bottom membrane 501 is provided in the culture space 101 are not limited particularly. Further, the lid 140 is covered on tops of the inserts 500, and in a state where the lid 140 is covered thereon, one end of each channel connection protrusion 141 for the injection of the culture media is located inside the culture space 101 (whereas the other end of each channel connection protrusion 141 is connected to the culture media feed channel). In this case, one end of each channel connection protrusion 141 is located toward the peripheral area of the bottom membrane 501 when viewed on the plane to thus prevent the culture media from being injected directly into the bottom membrane 501. Desirably, one of each channel connection protrusion 141 can be bent by means of a force applied by the user, and further, it is made of a material capable of keeping the bent state so that one end of each channel connection protrusion 141 can be changed in direction arbitrarily by the user. This is to prevent the culture media from being directly dropped to the center of the bottom membrane 501 while the cells are cultured.

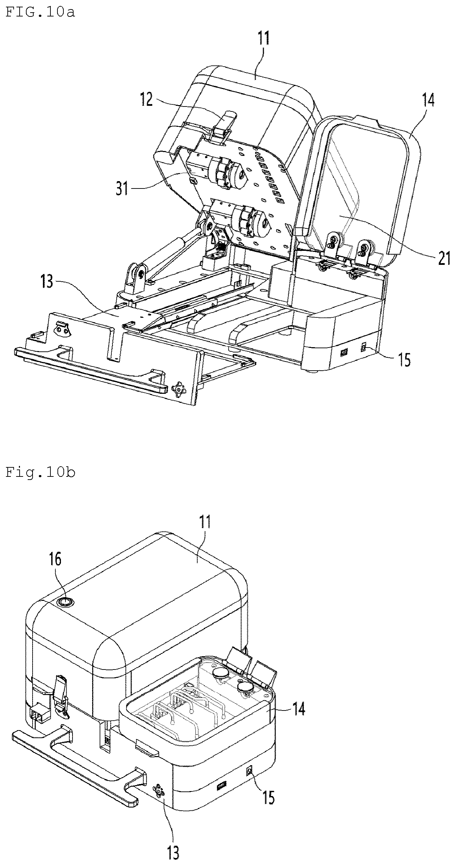

FIGs. 10a and 10b show the components of the cell culture device 1 according to an embodiment of the present invention. According to the embodiment of the present invention, the cell culture device 1 includes the cartridge 100, a pressing part 21 for applying an external force to the culture media adjusting part 110 of the cartridge 100, pumps 31 for continuously feeding the culture media, and a controller (not shown) for controlling overall operating states of the cell culture device 1. Further, the cell culture device 1 includes additional components as shown in FIGs. 10a and 10b. In detail, the cell culture device 1 includes a cap 11 for covering the culture media adjusting part 110 and receiving the pressing part 21 and the pumps 31 therein, a locking clip 12 for fixing the cap 11 after the cap 11 has been closed, a cartridge drawer 13 for slidingly coupling the cartridge 100 to the interior of the cell culture device 1 after the cartridge 100 has been located thereon, a culture space cap 14 for covering the culture spaces 101, and a power connector 15 for supplying power to the cell culture device 1. In this case, the cartridge drawer 13 is designed to be slidable, as shown, and further, means for fastening the cartridge drawer 13 and the cartridge 100 to each other are additionally provided to allow the cartridge 100 to be accurately fixed in position. Furthermore, the culture space cap 14 is made of a transparent material so that cell culture can be observed with the naked eye or a camera.

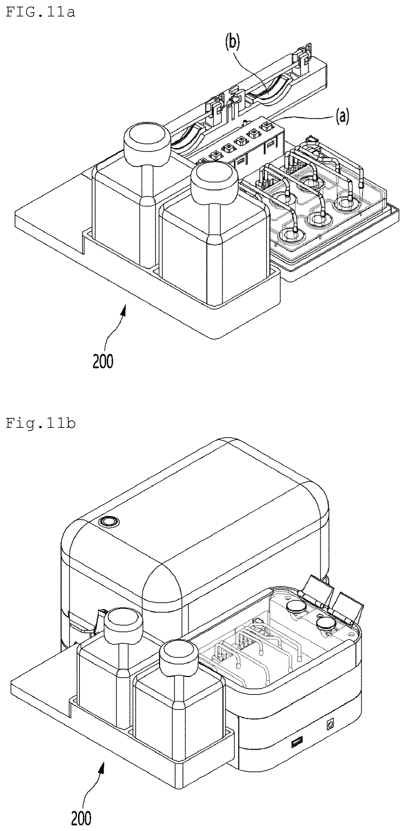

FIGs. 11a and 11b show a portion of the cartridge 100 that is brought into close contact with the pressing part 21 or the pumps 31 when the cartridge 100 is covered with the cap 11. As shown, the pressing part 21 is located above the culture media adjusting part 110, and the plurality of cylinders as shown in FIGs. 5 and 6 are selectively pressed against the pressing part 21 to allow the injection of the culture media to be adjusted. Further, the pumps 31 are located above the culture media feed channels 120 and the culture media feed channel support stands 121, and the pumps 31 engage with the culture media feed channel support stands 121 having the semicircular guide grooves and operate to allow the culture media to be fed to the culture media adjusting part 110.

FIGs. 11a and 11b shows an example wherein the culture media storage part 200 is additionally provided for the cartridge 100 and the cell culture device 1, and the culture media storage part 200 is located on the outside of the cell culture device 1 in such a way as to be simply replaceable so that the cell culture can be easily performed by the user.

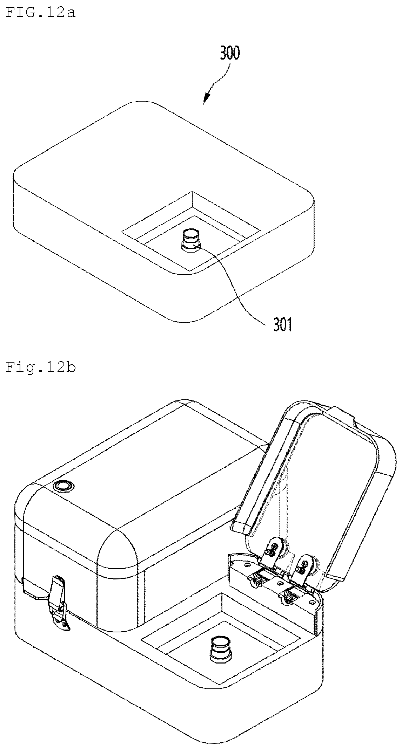

FIGs. 12a and 12b show a monitoring part 300 for monitoring growth of the cells in the culture spaces 101 of the cartridge 100. As shown, the monitoring part 300 is designed to basically have the shape of a hexahedron so that the cell culture device 1 is placed on top thereof and includes a camera 301 headed upwards to acquire images for the cell culture on the bottoms of the culture spaces 101. Further, lamps are provided around the camera 301 to emit light with given wavelengths so that more vivid cell images can be acquired when the culture spaces 101 are imaged.

While the present invention has been described with reference to the particular illustrative embodiments, it is not to be restricted by the embodiments but only by the appended claims. It is to be appreciated that those skilled in the art can change or modify the embodiments without departing from the scope and spirit of the present invention.

1 Cell culture device, 11 Cap, 12 Locking clip, 13 Cartridge drawer, 14 Culture space cap, 15 Power connector, 21 Pressing part, 31 Pump, 100 Cartridge, 101 Culture space, 102 Tray, 103 Tray stand, 110 Culture media adjusting part, 1121, 1123 Cylinder, 11211, 11231 Spring supporter, 11212, 11232 Tube compressor, 120 Culture media feed channel, 121 Culture media feed channel support stand, 122 Culture media feed channel support plate, 123 Culture media feed pipe, 130 Culture media injection channel, 131 Culture media discharge channel, 140 Lid. 141 Channel connection protrusion, 142 Lid fixing shaft, 143 Channel fixing stand, 200 Culture media storage part, 300 Monitoring part, 301 Camera

## Claims

1. A cartridge for adherent cell culture, comprising:
one or more culture spaces;
a culture media adjusting part for adjusting the injection of culture media into the culture spaces;
culture media feed channels for moving the culture media to the culture media adjusting part from the outside; and
culture media injection channels extending from the culture media adjusting part to move the culture media to the culture spaces,
wherein the culture media adjusting part comprises a connector adapted to branch the culture media fed from the culture media feed channels into the culture media injection channels.

2. The cartridge according to claim 1, further comprising actuators adapted to press the culture media injection channels made of a flexible material so that the injection of the culture media is controlled.

3. The cartridge according to claim 2, further comprising a lid for covering a whole of the culture spaces so that the culture media are injected into the culture spaces by means of the culture media injection channels passing through the lid.

4. The cartridge according to claim 3, wherein the lid is designed to receive an external force from the culture media injection channels passing through tops of the lids, and the culture media injection channels apply the external force to the lid so that the culture spaces are closed with the lid.

5. A device for automating cell culture, comprising:
a cartridge detachably mounted thereon and comprising:
one or more culture spaces;
a culture media adjusting part for adjusting the injection of culture media into the culture spaces;
culture media feed channels for moving the culture media to the culture media adjusting part from the outside; and
culture media injection channels extending from the culture media adjusting part to move the culture media to the culture spaces;
a pressing part for applying an external force to the culture media adjusting part; and
a controller for controlling the pressing part.
